# EUROPEAN PATENT APPLICATION

(11) **EP 2 281 551 A1**
(43) Date of publication of application: **09.02.2011**
(21) Application number: 09750562.2
(22) Date of filing: 19.05.2009
(51) Int. Cl.: A61K 8/66, A61K 8/20, A61K 8/23, A61K 8/25, A61Q 7/00, A61Q 19/00

(54) **METHOD FOR GROWING OR NOURISHING HEAD HAIR**

(30) Priority: 19.05.2008 JP 2008130512; 20.05.2008 JP 2008131893
(71) Applicant: TOWA Enzyme Co., Ltd., Hiratsuka-shi Kanagawa 254-0064 (JP); Thales Inc., Shinjuku-ku Tokyo 161-0033 (JP)
(72) Inventor: MIYAZAKI Kazuo, Hiratsuka-shi Kanagawa 254-0064 (JP); FUJIKAWA Susumu, Tokyo 161-0033 (JP)
(74) Representative: Adam, Holger
(86) International application number: PCT/JP2009/059187
(87) International publication number: WO 2009/142200

(57) **Abstract**

A hair growth method includes repeatedly washing hair using a washing agent that includes a microbubble washing composition and microbubbles, the microbubble washing composition including a protease and a lipase. The hair growth method exhibits an excellent hair growth effect.

## Description

### TECHNICAL FIELD

The present invention relates to a hair growth method that includes washing hair using a washing agent that includes a microbubble washing composition including a protease and a lipase, and microbubbles.

### BACKGROUND ART

The number of people who are troubled with hair loss, thinning hair, and the like has increased year by year. Young people have increasingly suffered from hair loss in a modem stress society. Therefore, development of a hair growth method that achieves a high hair growth effect has been desired.

For example, a method that uses a hair growth agent including 6-benzylaminopurine (6-benzyladenine) and a derivative thereof (Patent Document 1), a method that uses a skin cosmetic preparation (Patent Document 2), a method that uses a hair growth agent including an extract of *Coleus forskohlii* (Patent Documents 3 and 4), a hair/scalp washing agent that contains an anionic surfactant, a percutaneous absorption accelerator, and a bactericidal component, a percutaneous absorption accelerator, and a method of promoting percutaneous absorption of an active ingredient of a hair growth agent that includes washing hair using the hair/scalp washing agent before using a hair growth agent that contains an active ingredient (Patent Document 5), and the like have been known as a method that aims at preventing skin aging and activating the cells.

A hair growth method that achieves a high hair growth effect has been proposed in the field of hair growth agents. However, a sufficient hair growth effect has not been achieved.

A method that washes the pores in the scalp has been employed so that a medicine easily reaches the roots of the hair, and exhibits a sufficient hair growth effect. Specifically, if the pores in the scalp are blocked with sebum, a medicine cannot reach the roots of the hair, and does not exhibit a sufficient hair growth effect.

For example, Patent Document 6 discloses a hair washing method that includes covering a head with a washing cap, discharging a washing agent into the washing cap, and scrubbing the scalp with a brush or the like inserted into the washing cap. In this case, however, the pores cannot be sufficiently washed, and the scalp may be damaged when scrubbing the scalp with a brush.

Patent Document 7 discloses a hair washing method that utilizes a hair washing apparatus that includes a discharge section that discharges a washing agent into a washing cap that covers a head, and a recovery section that recovers the washing agent discharged from the washing cap, wherein the washing agent recovered by the recovery section is discharged from the discharge section, mixed with microbubbles, and circulated. Patent Document 8 discloses a microbubble washing method that washes a human body or an animal using a washing agent that includes a microbubble washing composition containing a protease and a lipase, and microbubbles. Patent Document 8 discloses that the washing method disclosed in Patent Document 8 exhibits an effect of reliably removing sebum and old keratin adhering to the surface of the pores of a human body or an animal and wastes inside the pores within a short time, and keeping the skin clean for a long time, an effect of preventing skin diseases, and an effect of accelerating the healing of skin disease. However, these documents do not disclose that a hair growth effect is obtained by combining microbubbles with a given washing agent.

### RELATED-ART DOCUMENT

### PATENT DOCUMENT

Patent Document 1: JP-A-05-320028
Patent Document 2: JP-A-07-233037
Patent Document 3: JP-A-08-176005
Patent Document 4: JP-A-09-157138
Patent Document 5: JP-A-2007-31376
Patent Document 6: JP-A-2003-210240
Patent Document 7: JP-A-2007-252434
Patent Document 8: JP-A-2008-94726

### DISCLOSURE OF THE INVENTION

### PROBLEMS TO BE SOLVED BY THE INVENTION

The present invention was conceived in view of the above situation. An object of the present invention is to provide a hair growth method that exhibits an excellent hair growth effect.

### MEANS FOR SOLVING THE PROBLEMS

In order to achieve the above object, the inventors repeatedly washed hair every given period (about twice a week) using a washing agent that includes a microbubble washing composition including a protease and a lipase, and microbubbles by applying the method disclosed in Patent Document 8. Surprisingly, the inventors found that a hair growth effect can be obtained without using a hair growth agent or a hair tonic. The inventors also found that a more excellent hair growth effect can be obtained by applying a commercially available hair growth agent or hair tonic to the hair after washing the hair using the washing agent. These findings have led to the completion of the present invention.

Specifically, the present invention provides the following hair growth method (see (1) to (7)).
(1) A hair growth method comprising repeatedly washing hair using a washing agent that includes a microbubble washing composition and microbubbles, the microbubble washing composition including a protease and a lipase.
(2) The hair growth method according to (1), wherein the microbubble washing composition includes the protease in an amount of 0.01 to 0.5 wt% based on the total amount of the microbubble washing composition, and includes the lipase in an amount of 0.1 to 1.0 wt% based on the total amount of the microbubble washing composition.
(3) The hair growth method according to (1) or (2), wherein the microbubble washing composition does not include a surfactant.

(4) The hair growth method according to any one of (1) to (3), wherein the microbubble washing composition further includes at least one alkali metal salt selected from the group consisting of alkali metal carbonates, alkali metal hydrogen carbonates, alkali metal sulfates, alkali halides, and alkali metal salts of boron.
(5) The hair growth method according to (4), wherein the microbubble washing composition includes the alkali metal salt in an amount of 80 to 99.5 wt% based on the total amount of the microbubble washing composition.
(6) The hair growth method according to any one of (1) to (5), wherein the hair is washed 1 to 3 times a week.
(7) The hair growth method according to any one of (1) to (6), further comprising applying a given amount of a hair growth agent or a hair tonic to the hair after washing the hair.

### EFFECTS OF THE INVENTION

According to the present invention, a hair growth effect can be obtained without using a hair growth agent or a hair tonic. A more excellent hair growth effect can be obtained by applying a commercially available hair growth agent or hair tonic to the hair after washing the hair.

### BRIEF DESCRIPTION OF THE DRAWINGS

FIG. 1 is a view showing the configuration of a hair washing apparatus used in the examples.
FIG. 2 shows a photograph of the hair (scalp) of a male subject with time (Example 1).
FIG. 3 shows a photograph of the hair (scalp) of a male subject with time (Example 2).
FIG. 4 shows a photograph of the hair (scalp) of a male subject with time (Example 3).
FIG. 5 shows a photograph of the hair (scalp) of a male subject with time (Example 4).

### DESCRIPTION OF EXEMPLARY EMBODIMENTS

A hair growth method according to one embodiment of the present invention includes repeatedly washing hair using a washing agent that includes a microbubble washing composition and microbubbles, the microbubble washing composition including a protease and a lipase.

The protease included in the microbubble washing composition (hereinafter may be referred to as "composition") according to one embodiment of the present invention is an enzyme that catalyzes hydrolysis of a peptide bond.

The protease has a specific optimum pH, and is classified as an acidic proteinase, a neutral proteinase, or an alkaline proteinase. Any of these proteases may be used in the present invention without specific limitations.

Examples of the protease include, but are not limited to, chymotrypsin, subtilisin, pepsin, cathepsin D, thermolysin, papain, caspase, bromelain, actinidin, ficin, trypsin, pancreatin, and the like.
These proteases may be used either individually or in combination.

Specific examples of the protease include Protin AC10F (manufactured by Daiwa Kasei K.K.), Savinase, Alcalase, Esperase, Durazyme (manufactured by Novozymes), Maxapem, Maxatase (manufactured by Gist-brocades), Bioplaze (manufactured by Nagase Biochemicals, Ltd.), and the like.

It is preferable to use at least pancreatin in order to obtain excellent washing effects. It is more preferable to use pancreatin and another protease in combination.

The amount of protease used is not particularly limited. The protease is preferably used in an amount of 0.01 to 0.5 wt%, and more preferably 0.2 to 0.3 wt%, based on the total amount of the composition.

The lipase included in the composition according to one embodiment of the present invention is an enzyme that hydrolyzes fats into glycerol and a fatty acid.

Examples of the lipase include, but are not limited to, a lipase derived from *Rhizopus arrhizus,* a lipase derived from *Aspergillus niger,* and a lipase derived from a mold such as *Rhizopus delemar;* a lipase derived from yeast such as *Candida cylindracea;* a lipase derived from a bacteria such as *Pseudomonas;* a lipase that is present in the digestive tract tissues of a ruminant during a lactation period, such as pregastric lipase or oral lipase; pig liver lipase; and the like.
These lipases may be used either individually or in combination. It is preferable to use two or more lipases in order to obtain more excellent washing effects.

Specific examples of the lipase include Sumizyme NLS (manufactured by Shin-Nihon Kagaku Kogyo, Co., Ltd.), Lipase M "Amano" 10, Lipase M "Amano" 10, Lipase G "Amano" 50, Lipase F-AP15, Lipase AY "Amano" 30G, Lipase R "Amano" G, Lipase T "Amano", Lipase MER "Amano" (manufactured by Amano Enzyme Inc.), Picantase R8000, Picantase A (manufactured by Robin), Toyozyme and LIP (manufactured by Toyobo Co., Ltd.), Lilipase A-10FG, Lilipase AF-5 (manufactured by Nagase ChemteX Corporation), Grindamyl EXEL639 (manufactured by Danisco Culter Japan), Clear-Lens Lipo, Liporaze, Lipex, Lipozyme, Resinase, Paratase, Greasex, Lipopan, Novozyme 435, Lecitase (manufactured by Novozymes), Lipase MY, Lipase OF, Lipase PL, Lipase QLM, Lipase AL, Phospholipase D (manufactured by Meito Sangyo Co., Ltd.), Enzylon AKG (manufactured by Rakuto Kasei Industrial Co., Ltd.), Phospholipase Al (manufactured by Sankyo Lifetech Co., Ltd.), Lipomod 699L, Lysomax PF (manufactured by Genencor Kyowa Co., Ltd.), and the like.

The lipase is normally used in an amount of 0.1 to 1.0 wt%, and preferably 0.3 to 0.8 wt%, based on the total amount of the composition.

It is preferable that the composition according to the present invention does not include a surfactant from the viewpoint of obtaining more excellent washing effects. When the composition does not include a surfactant, the composition does not irritate the eyes even if the composition gets in the eyes when washing hair.

It is preferable that the composition according to the present invention further include an alkali metal salt in addition to the protease and the lipase.

The alkali metal salt improves the washing effects, softens water (warm water), and functions as a filler.

Examples of the alkali metal salt include, but are not limited to, alkali metal carbonates such as sodium carbonate and potassium carbonate; alkali metal hydrogen carbonates such as sodium hydrogen carbonate and potassium hydrogen carbonate; alkali metal sulfates such as sodium sulfate and potassium sulfate; alkali halides such as sodium chloride and potassium chloride; alkali metal salts of boron such as sodium tetraborate (borax) and sodium borate; alkali metal silicates such as sodium silicate; alkali metal sulfides such as sodium sulfide; alkali metal nitrates such as sodium nitrate; alkali metal phosphates such as sodium phosphate and sodium dihydrogen phosphate; thiosulfates of an alkali metal such as sodium thiosulfate; and the like.
These alkali metal salts may be used either individually or in combination.

It is preferable that the composition according to the present invention include at least one alkali metal salt selected from the group consisting of alkali metal carbonates, alkali metal hydrogen carbonates, alkali metal sulfates, alkali halides, and alkali metal salts of boron. It is more preferable that the composition include at least one alkali metal salt selected from the group consisting of sodium hydrogen carbonate, sodium sulfate, and borax. It is particularly preferable that the composition include sodium hydrogen carbonate, sodium sulfate, and borax.

The alkali metal salt is normally used in an amount of 80 to 99.5 wt%, and preferably 95 to 99 wt%.
When the washing composition according to the present invention includes sodium hydrogen carbonate, sodium sulfate, and borax, sodium hydrogen carbonate is preferably used in an amount of 10 to 35 wt%, and particularly preferably 20 to 30 wt%, based on the total amount of the composition, sodium sulfate is preferably used in an amount of 30 to 70 wt%, and particularly preferably 40 to 60 wt%, based on the total amount of the composition, and borax is preferably used in an amount of 10 to 35 wt%, and particularly preferably 20 to 30 wt%, based on the total amount of the composition.

It is preferable that the composition according to the present invention further include a silica-based desiccant.
The desiccant stabilizes the potency of the protease and the lipase. Examples of the silica-based desiccant include Sylysia (porous fine silica powder) and the like.
The silica-based desiccant is normally used in an amount of 0.01 to 1 wt%, and preferably 0.1 to 0.2 wt%.

The composition according to the present invention may optionally further include an additional component.
Examples of the additional component include inorganic salts other than the above alkali metal salts, a perfume, a coloring agent, an antiseptic agent, an antibacterial agent, a tackiness agent, a therapeutic component, and other additives normally used for a washing agent.

Examples of inorganic salts other than the alkali metal salts include magnesium salts such as magnesium carbonate and magnesium sulfate; calcium salts such as calcium carbonate, calcium nitrate, calcium thiosulfate, and calcium hydrogen phosphate; alum; and the like. The amount of inorganic salts may be appropriately determined insofar as the effects of the microbubble washing composition are not impaired.

Examples of the perfume include essential oils such as abies oil, angelica oil, anise oil, copaiba balsam, basil oil, bay oil, bergamot oil, birch oil, rosewood oil, cajabute oil, cassia oil, acacia oil, cedar wood oil, chamomile oil, cinnamon oil, cinnamon leaf oil, citronella oil, elemi oil, eucalyptus oil, geranium oil, white-cedar leaf oil, cypress oil, lavandin oil, lavender oil, lemon oil, mint oil, neroli oil, nutmeg oil, oakmoss oil, ocotea oil, patchouli oil, palmarosa oil, plai oil, rose oil, rosemary oil, sandalwood oil, vetiver oil, and ylang ylang oil; synthetic perfumes such as synthetic musk (e.g., limonene, terpinolene, p-cymene, 9-decenol, mugol, myrcenol, borneol, vetiverol, t-butylcyclohexanol, anisole, anethole, safrole, citral, citronellal, cinnamaldehyde, anise aldehyde, cyclamen aldehyde, citral, acetophenone, benzophenone, acetonaphthone, nerone, and nitromusk), geranyl acetate, bornyl acetate, phenylethyl acetate, myrcenyl acetate, methyl benzoate, methyl cinnamate, methyl anthranilate, synthetic oakmoss, and coumarin; and the like.
The perfume is normally used in an amount of 0 to 0.5 wt%, and preferably 0.1 to 0.3 wt%.

As the coloring agent, a dye, a natural pigment, an inorganic dye, and the like may be used.
Examples of the dye include Red No. 2, Red No. 3, Red No. 102, Red No. 104-1, Red No. 105-1, Red No. 106, Red No. 213, Red No. 227, Red No. 230-1, Yellow No. 4, Yellow No. 5, Yellow No. 201, Yellow No. 201-1, Yellow No. 203, Orange No. 205, Green No. 3, Green No. 201, Green No. 204, Blue No. 1, Blue No. 2, Blue No. 205, Violet No. 201, Brown No. 201, and the like.

Examples of the natural pigment include a chlorophyll, a gardenia, a flavonoid, a carotinoide, a quinone, and a riboflavin.

Examples of the inorganic dye include titanium dioxide, talc, kaolin, mica, magnesium silicate, silicic anhydride, calcium carbonate, magnesium carbonate, and the like.
These coloring agents may be used either individually or in combination.
The coloring agent is normally used in an amount of 0 to 0.5 wt%, and preferably 0.1 to 0.2 wt%.

Examples of the antiseptic agent include sodium benzoate, methyl p-hydroxybenzoate, ethyl p-hydroxybenzoate, propyl p-hydroxybenzoate, butyl p-hydroxybenzoate, isobutyl p-hydroxybenzoate, benzyl alcohol, benzalkonium chloride, and the like.

Examples of the tackiness agent include hydroxyethylcellulose, hydroxypropylmethylcellulose, methylcellulose, xanthan gum, polyethylene glycol distearate, polyethylene glycol monostearate, and the like.

The composition according to the present invention may be produced by an arbitrary method. For example, a mixture of the protease, the lipase, and other optional components is homogenously stirred (mixed) using a stirrer such as a Vert-o-Mix, a Nauta mixer, a universal mixer/stirrer, a ribbon mixer, a V-shaped mixer, or the like to prepare a powder.

The composition according to the present invention can reliably remove sebum and old keratin adhering to the surface of the pores of the skin within a short time, and keep the skin clean for a long time.
Since the composition according to the present invention does not damage hair due to low irritation properties, the composition can be safely used for a person having a sensitive skin, an elderly person having a weak skin, or a human suffering from skin diseases.

The hair growth method according to the present invention includes repeatedly washing hair using a washing agent that includes the composition according to the present invention and microbubbles.

Specifically, the hair growth method according to the present invention includes adding an appropriate amount of the composition according to the present invention to clean water (or warm water) to prepare a washing agent, introducing microbubbles into the washing agent, and repeatedly washing hair using the washing agent.

The expression "repeatedly washing hair" used herein refers to washing hair at least twice at a given interval. The expression "at a given interval" used herein refers to washing hair once every three days. The given interval is not particularly limited. An interval of about 1 to 3 times a week is normally employed according to the present invention. Hair is repeatedly washed until the desired hair growth effect is achieved. The period is normally from about several weeks to about one year. A sufficient hair growth effect can be obtained by repeatedly washing hair for such a period at the above intervals.

When producing the washing agent, the composition according to the present invention is normally used in an amount of 1 to 10 g, and preferably 3 to 7 g, per 6 to 10 liters of water.

The microbubble washing time is usually about one minute to several tens of minutes, although the time varies depending on the type and concentration of the microbubble washing composition, the washing portion, and the like.
The washing temperature is not particularly limited, but is preferably 20 to 40°C from the viewpoint of achieving excellent washing effects and relaxation.

Since the method according to the present invention can reliably remove sebum and old keratin adhering to the surfaces of the pores within a short time and keep the skin clean for a long time, an excellent hair growth effect can be obtained by bringing out the original hair growth effect.

Since the washing agent including the microbubbles does not adversely affect the cells of a human body, does not damage hair, and is environmentally friendly, the washing agent can be used safely.

The microbubbles are minute bubbles having a diameter of 50 µm or less, and are reduced in size and disappear (collapse) in water, differing from normal bubbles. The microbubbles remove dirt due to impact waves that occur when the microbubbles break. The microbubbles carry negative ions, and disappear in water while bonding to dirt (positive ions) and floating, so that the dirt floats. When using the washing agent including the microbubbles to wash a human body or an animal, minute microbubbles can reach the interior of the pores to adsorb and remove wastes.

A washing agent including the microbubbles is normally produced using a microbubble generator described below. Since water has a high surface tension, it is impossible to produce bubbles having a diameter of 100 µm or less by normal bubbling.

The microbubble generator is not particularly limited insofar as microbubbles can be produced. A known microbubble generator may be used. Examples of the microbubble generator include a device that produces microbubbles in a venturi tube (tube with a narrow constriction) or a shower head including an orifice plate (doughnut plate with a center opening) (e.g., JP-A-2006-116518 and Japanese Patent Application No. 2006-77553); a device that produces microbubbles by injecting a liquid mixed with a gas into a main pipe disposed in a liquid, and causing the liquid to collide with a collision wall disposed downstream of the main pipe (e.g., JP-A-2005-334869); a device that produces microbubbles by injecting pressurized air into water through a mesh member or a porous plate with a minute pore size using a pressurized air supply source such as an air pump (e.g., JP-A-2006-68631); a device that produces microbubbles by producing a whirling water stream, and shearing air using the water stream (e.g., JP-A-2003-126665); and the like. When washing human hair, it is preferable to use the microbubble generation device disclosed in Japanese Patent Application No. 2006-77553.

Examples of the method of washing hair using the washing agent including the microbubbles include (i) a method that includes placing a washing agent obtained by adding an appropriate amount of the composition to clean water (or warm water) in a container such as a tank, introducing the washing agent into a microbubble generator by an appropriate method, introducing microbubbles into the washing agent using the microbubble generator, and washing hair while discharging the washing agent including the microbubbles from a shower nozzle; (ii) a method that includes placing clean water (or warm water) in a washing bath, adding an appropriate amount of the composition according to one embodiment of the present invention to the washing bath to prepare a washing agent, introducing water (or warm water) including microbubbles into the washing agent from a nozzle connected to a microbubble generator to prepare a washing agent including microbubbles, and washing hair using the resulting washing agent; and the like. These methods may be used in combination.

When using the method (i), it is preferable to circulate the washing agent while removing contaminants and the like using a filter in order to efficiently utilize the washing agent.

After washing the hair, the washing agent is removed by rinsing the washing agent out of the hair with water or warm water, for example.

Moreover, braided hair such as dreadlocks can be directly washed using the washing agent that includes the microbubble washing composition including a protease and a lipase, and microbubbles.

### EXAMPLES

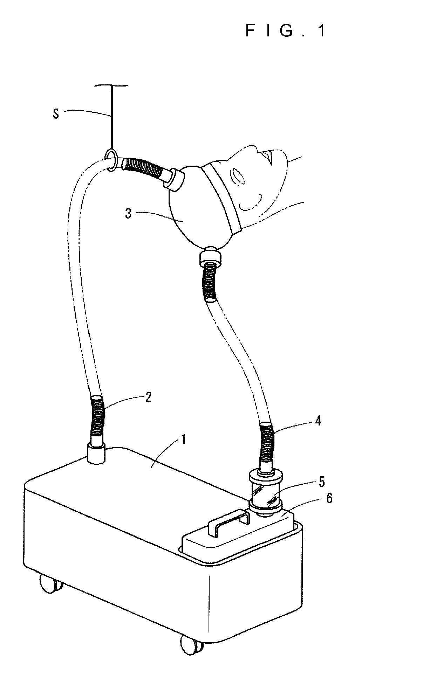

The present invention is further described below by way of examples. Note that the present invention is not limited to the following examples.
A hair washing apparatus shown in FIG. 1 was used in the examples (this apparatus is disclosed in JP-A-2007-252434). The hair washing apparatus shown in FIG. 1 has the following configuration.

In FIG. 1, reference numeral 1 indicates a main body, reference numeral 2 indicates a discharge tube, reference numeral 3 indicates a washing cap, reference numeral 4 indicates a recovery tube, reference numeral 5 indicates a filter unit, reference numeral 6 indicates a cartridge tank, and reference numeral 7 indicates a nozzle unit.
As shown in FIG. 1, the main body 1 is charged with warm water that is supplied from the cartridge tank 6 and includes the microbubble washing composition that includes the protease and the lipase. Warm water (30 to 40°C) including the washing composition is discharged from the main body 1 together with microbubbles, and supplied to the washing cap 3 through the discharge tube 2 to wash the hair. The washing agent is recovered from the washing cap 3 into the filter unit 5 through the recovery tube 4. The filter unit 5 removes hair and dirt (e.g., dandruff) from the washing agent. The washing agent then enters the cartridge tank 6. The discharge tube 2 is suspended by the suspension tool S at an appropriate position.

### (Example 1)

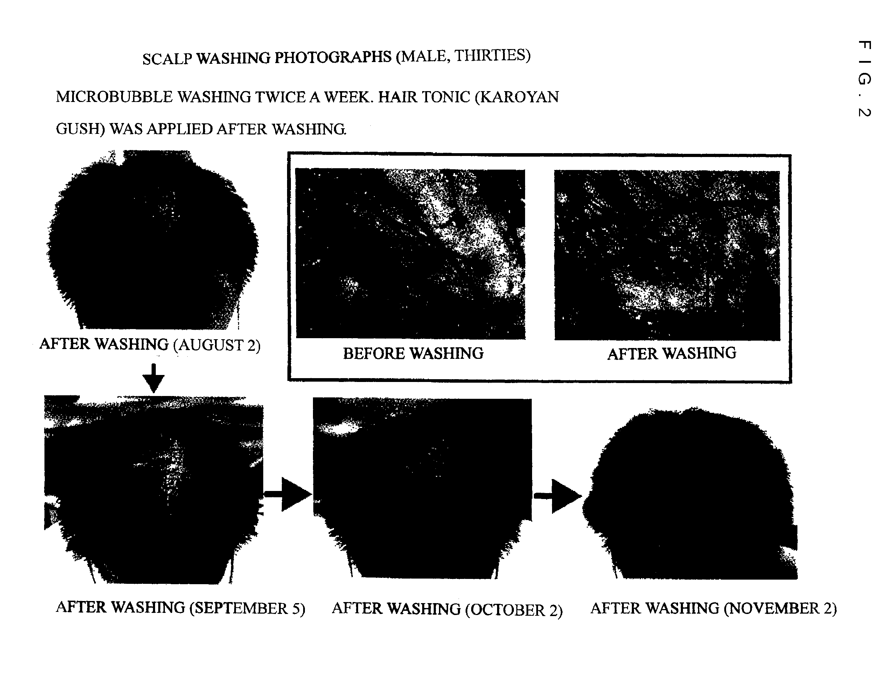

The hair (scalp) of a subject (male, thirties) was washed twice a week using the hair washing apparatus shown in FIG. 1 over about five months. The washing time per operation was about 15 minutes. After washing the hair, 2 ml of a commercially available hair tonic ("Karoyan Gush" manufactured by Daiichi Sankyo Healthcare Co., Ltd.) was applied to the hair. FIG. 2 shows a photograph of the hair of the subject with time. As shown in FIG. 2, a significant hair growth effect was observed by washing the hair (scalp) twice a week over about five months.

### (Example 2)

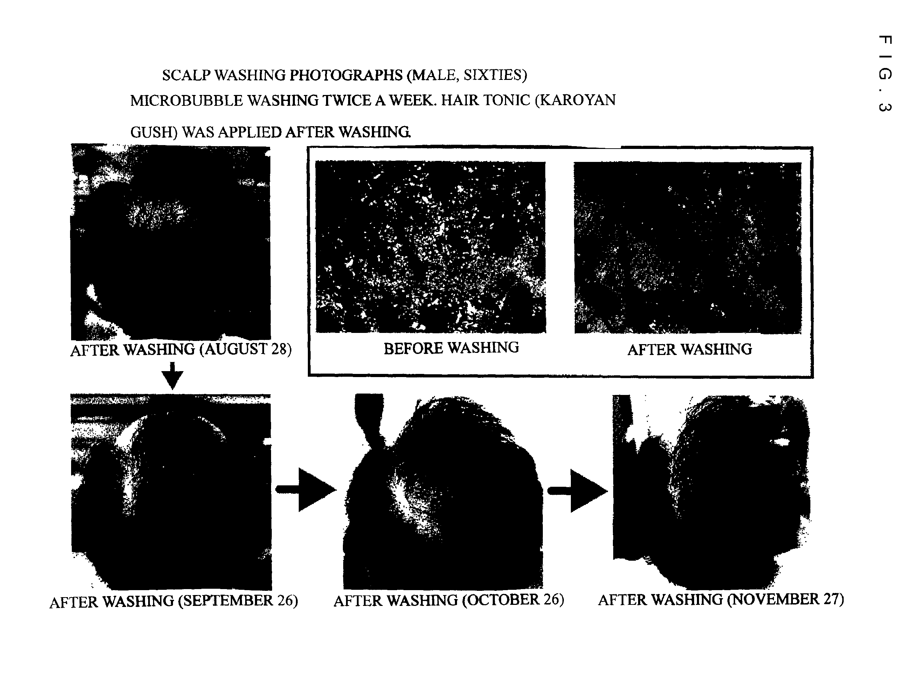

The hair (scalp) of a subject (male, sixties) was washed twice a week using the hair washing apparatus shown in FIG. 1 over about five months. The washing time per operation was about 15 minutes. After washing the hair, 2 ml of a commercially available hair tonic ("Karoyan Gush" manufactured by Daiichi Sankyo Healthcare Co., Ltd.) was applied to the hair. FIG. 3 shows a photograph of the hair of the subject with time. As shown in FIG. 3, a significant hair growth effect was observed by washing the hair (scalp) twice a week over about five months.

### (Example 3)

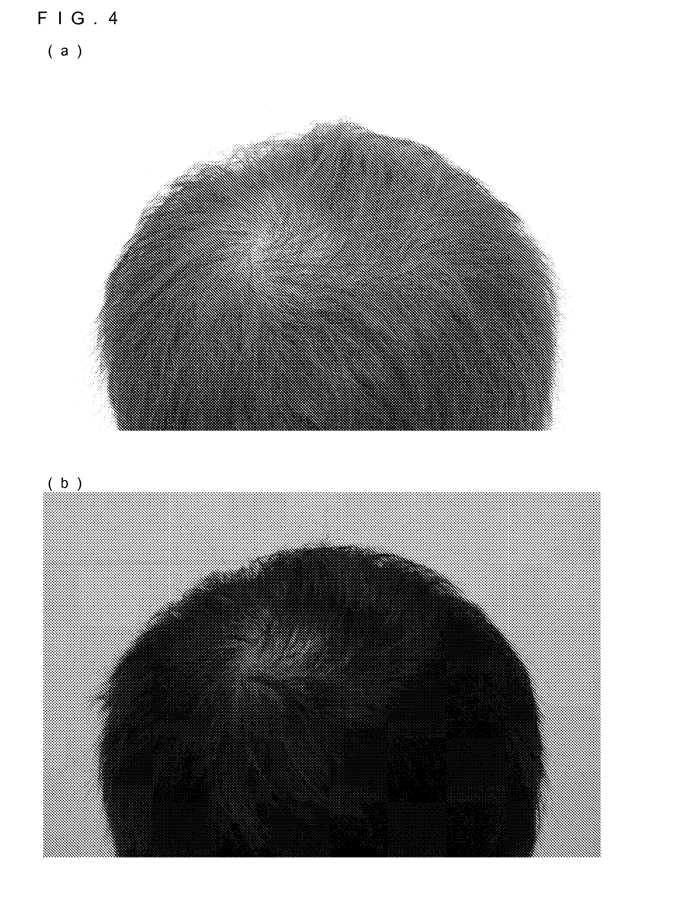

The hair (scalp) of a subject (male, fifties) was washed twice a week using the hair washing apparatus shown in FIG. 1 over about seven months. The washing time per operation was about 15 minutes. After washing the hair, 2 ml of a commercially available hair tonic ("Karoyan Gush" manufactured by Daiichi Sankyo Healthcare Co., Ltd.) was applied to the hair. FIG. 4 shows a photograph of the hair of the subject with time. In FIG. 4, (a) indicates a photograph taken on September 24, 2008, and (b) indicates a photograph taken on April 3, 2009. As shown in FIG. 4, a significant hair growth effect was observed by washing the hair (scalp) twice a week over about six months.

### (Example 4)

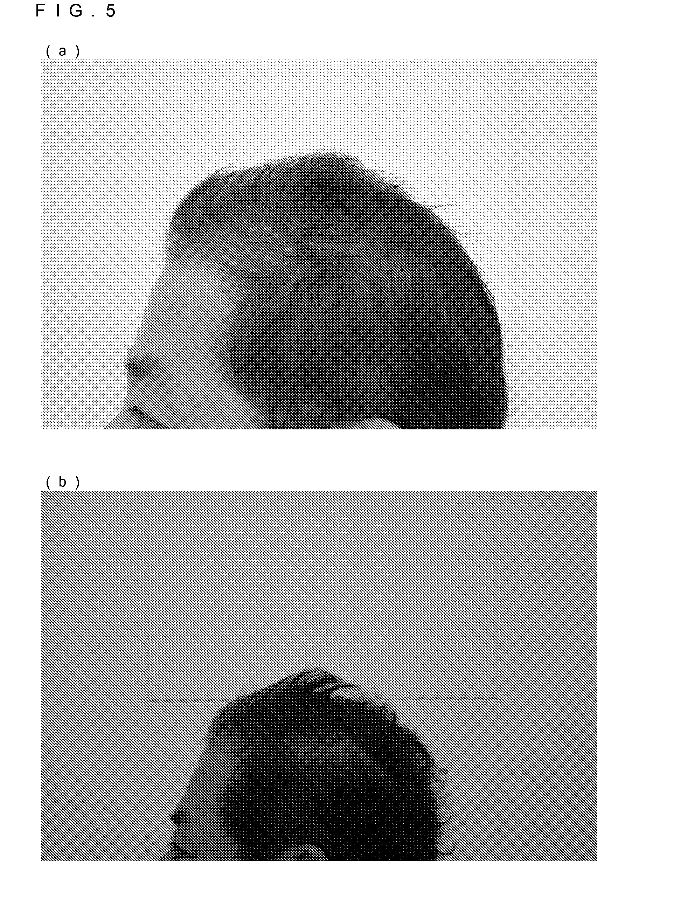

The hair (scalp) of a subject (male, thirties) was washed twice a week using the hair washing apparatus shown in FIG. 1 over about seven months. The washing time per operation was about 15 minutes. After washing the hair, 2 ml of a commercially available hair tonic ("Karoyan Gush" manufactured by Daiichi Sankyo Healthcare Co., Ltd.) was applied to the hair. FIG. 5 shows a photograph of the hair of the subject with time. In FIG. 5, (a) indicates a photograph taken on September 12, 2008, and (b) indicates a photograph taken on April 3, 2009. As shown in FIG. 5, a significant hair growth effect was observed by washing the hair (scalp) twice a week over about six months.

### EXPLANATION OF SYMBOLS

1: main body, 2: discharge tube, 3: washing cap, 4: recovery tube, 5: filter unit, 6: cartridge tank, 7: nozzle unit

## Claims

1. A hair growth method comprising repeatedly washing hair using a washing agent that includes a microbubble washing composition and microbubbles, the microbubble washing composition including a protease and a lipase.

2. The hair growth method according to claim 1, wherein the microbubble washing composition includes the protease in an amount of 0.01 to 0.5 wt% based on the total amount of the microbubble washing composition, and includes the lipase in an amount of 0.1 to 1.0 wt% based on the total amount of the microbubble washing composition.

3. The hair growth method according to claim 1 or 2, wherein the microbubble washing composition does not include a surfactant.

4. The hair growth method according to any one of claims 1 to 3, wherein the microbubble washing composition further includes at least one alkali metal salt selected from the group consisting of alkali metal carbonates, alkali metal hydrogen carbonates, alkali metal sulfates, alkali halides, and alkali metal salts of boron.

5. The hair growth method according to claim 4, wherein the microbubble washing composition includes the alkali metal salt in an amount of 80 to 99.5 wt% based on the total amount of the microbubble washing composition.

6. The hair growth method according to any one of claims 1 to 5, wherein the hair is washed 1 to 3 times a week.

7. The hair growth method according to any one of claims 1 to 6, further comprising applying a given amount of a hair growth agent or a hair tonic to the hair after washing the hair.
